# EUROPÄISCHE PATENTSCHRIFT

(11) **EP 2 379 138 B1**
(45) Veröffentlichungstag und Bekanntmachung des Hinweises auf die Patenterteilung: **31.10.2018**
(21) Anmeldenummer: 09782215.9
(22) Anmeldetag: 26.08.2009
(51) Int. Cl.: A61M 5/315

(54) **DOSIERVORRICHTUNG FÜR EINE INJEKTIONSVORRICHTUNG**
DOSING DEVICE FOR AN INJECTION DEVICE
DISPOSITIF DE DOSAGE POUR UN DISPOSITIF D'INJECTION

(30) Priorität: 22.12.2008 WO PCT/EP2008/011019
(43) Veröffentlichungstag der Anmeldung: 26.10.2011
(73) Patentinhaber: Tecpharma Licensing AG, 3401 Burgdorf (CH)
(72) Erfinder: BURREN, Stefan, CH-3150 Schwarzenburg (CH); HIRSCHEL, Jürg, CH-3007 Bern (CH); MARTINOIA, Samuel, CH-3052 Zollikofen (CH); MOSER, Ulrich, CH-3412 Heimiswil (CH); TSCHIRREN, Markus, CH-3400 Burgdorf (CH)
(86) Internationale Anmeldenummer: PCT/EP2009/060998
(87) Internationale Veröffentlichungsnummer: WO 2010/072427

(56) Entgegenhaltungen:
- EP-A1- 1 541 185
- WO-A1-2006/125329
- WO-A2-2004/078240
- DE-A1-102005 063 311
- US-A- 6 004 297
- US-A1- 2006 153 693

## Beschreibung

Die vorliegende Erfindung bezieht sich auf eine Dosiervorrichtung für eine Injektionsvorrichtung, insbesondere auf eine Dosiervorrichtung, mit welcher eine Dosis oder Menge einer aus oder mit Hilfe der Injektionsvorrichtung abzugebenden Substanz eingestellt werden kann.

Aus der WO 2006/125329 A1 der Anmelderin sind eine Dosiervorrichtung und eine Injektionsvorrichtung bekannt. Bezüglich des prinzipiellen Aufbaus einer Dosiervorrichtung und einer Injektionsvorrichtung wird auf diese Anmeldung Bezug genommen, welche vollumfänglich in die Beschreibung der vorliegenden Erfindung aufgenommen wird.

Aus der WO 2004/078240 A2 sind eine Dosiervorrichtung gemäß dem Oberbegriff des Anspruchs 1 2. und eine Injektionsvorrichtung bekannt, wobei die Dosiervorrichtung ein hülsenartiges Einstellelement (50) umfasst, welches über ein Kupplungselement (60) mit einem Vorschubelement (32) koppelbar ist. Das Vorschubelement (32) ist über eine Rückdrehsicherung (40) mit dem Gehäuse (2) der Vorrichtung verbunden, welche eine Rotation des Vorschubelements (32) relativ zum Gehäuse (2) nur in eine Richtung zulässt. Weiter verfügt die offenbarte Dosiervorrichtung über einen Begrenzungsmechanismus (64) zum Begrenzen einer maximal verabreichbaren Dosis.

Aus der EP 1541185 A1 sind ebenfalls eine Dosiervorrichtung und eine Injektionsvorrichtung bekannt. Die Dosiervorrichtung umfasst ein hülsenförmiges Einstellelement (30), welches über ein Kupplungselement (40) mit einem Vorschubelement (50) koppelbar ist. Das Vorschubelement (50) ist über eine Rückdrehsicherung (70) mit dem Gehäuse (20) verbunden, welche eine Rotation des Vorschubelements (50) relativ zum Gehäuse 20) nur in eine Richtung zulässt. Weiter verfügt die offenbarte Dosiervorrichtung über einen Begrenzungsmechanismus (34) zum Begrenzen einer maximal verabreichbaren Dosis. Die vorliegende Erfindung bezieht sich auf eine Dosiervorrichtung für eine Injektionsvorrichtung gemäss Anspruch 1 mit einem Betätigungselement, wie zum Beispiel einem in der Dosiervorrichtung oder in einem Gehäuse der Injektionsvorrichtung oder einem zum Beispiel fest, also zum Beispiel drehfest und/oder verschiebesicher, mit dem Gehäuse verbindbarem oder einsetzbarem Einsatz geführten und zum Beispiel damit im Gewindeeingriff stehenden Drehknopf oder Drehhülse, welcher zum Einstellen einer aus der Injektionsvorrichtung abzugebenden Dosis bewegt und zum Beispiel herausgeschraubt werden kann. Das Betätigungs- oder Einstellelement kann zum Einstellen der Dosis aus der Dosiervorrichtung zum Beispiel in proximale Richtung herausgeschraubt werden und zum Abgeben der Dosis wieder vollständig zurückgedreht oder eingeschraubt werden. Ein Vorschubelement, nämlich eine Gewindestange, ist in der Dosiervorrichtung gelagert und in einem Innengewinde der Dosiervorrichtung geführt. Die Gewindestange kann an ihrem distalen Ende zum Beispiel einen Flansch aufweisen, welcher auf einen Stopfen einer in der Injektionsvorrichtung enthaltenen Ampulle drückt und diesen Stopfen in die Ampulle hinein verschieben kann, um eine in der Ampulle enthaltene Substanz zu verdrängen und diese Substanz somit aus der Injektionsvorrichtung abzugeben. Das Betätigungselement und das Vorschubelement, also beispielsweise ein drehbarer Einstellknopf und eine Gewindestange, sind erfindungsgemäß zum Beispiel durch eine Klauenkupplung beim oder vor dem Ausschütten so koppelbar oder gekoppelt, dass eine Ausschüttbewegung des Betätigungselementes, also zum Beispiel eine Drehbewegung, direkt oder unmittelbar auf das Vorschubelement übertragen werden kann. Dabei kann die direkte oder unmittelbare Übertragung auch mittels eines oder mehrerer zwischengeschalteter Elemente, wie zum Beispiel mittels einer Kupplung, erfolgen. Vorzugsweise sind das Betätigungselement und das Vorschubelement zumindest während eines Ausschüttvorgangs verdrehsicher gekoppelt.

Die direkte oder unmittelbare Übertragung einer Bewegung und insbesondere einer Drehbewegung von einem Betätigungselement auf das Vorschubelement ermöglicht einen guten Kräftewirkungsgrad der Dosiervorrichtung. Beispielsweise ist es erfindungsgemäß nicht erforderlich, dass eine Drehbewegung des Betätigungselementes erst über einen oder mehrere Gewindeeingriffe auf das Vorschubelement übertragen wird. Bei der direkten Kopplung sind das Betätigungselement und das Vorschubelement vorzugsweise verdrehfest miteinander verbunden, so dass keine Über- oder Untersetzung der Drehbewegung vorliegt.

Das Betätigungselement ist über ein Kupplungselement oder eine Kupplungseinheit mit dem Vorschubelement gekoppelt. Das Kupplungselement kann zum Beispiel in Form einer Klauenkupplung ausgebildet sein und die Kopplung zwischen dem Betätigungselement und dem Vorschubelement so realisieren, dass während einer Einstellbewegung des Betätigungselementes das Betätigungselement und das Vorschubelement entkoppelt sind, also zum Beispiel das Betätigungselement unabhängig vom Vorschubelement zum Einstellen einer Dosis in eine Richtung und zur Dosiskorrektur in die Gegenrichtung frei gedreht werden kann. Nach dem Einstellen und optional dem Korrigieren einer Dosis wird mittels des Kupplungselementes das Betätigungselement mit dem Vorschubelement verdrehsicher gekoppelt, indem es zum Beispiel axial verschoben wird, da zum Beispiel in axialer Richtung ein Druck auf das Kupplungselement ausgeübt wird, zum Beispiel durch Drücken auf einen am proximalen Ende der Injektionsvorrichtung vorgesehene Knopf, und somit das zuvor vom Betätigungselement entkoppelte Kupplungselement mit dem Betätigungselement verdrehsicher gekoppelt wird. Das Kupplungselement ist vorzugsweise mit der Gewindestange zum Beispiel durch einen in eine Längsnut der Gewindestange eingreifenden Steg des Betätigungselementes (oder umgekehrt) verdrehsicher gekoppelt. Somit kann nach dem Schließen der Kupplung, also zum Beispiel dem bevorzugt verdrehsicheren Einkoppeln des Kupplungselements in das Betätigungselement, eine Drehbewegung des Betätigungselementes unmittelbar und ohne ein dazwischen liegendes Gewinde auf das Kupplungselement übertragen werden, welches sich zusammen mit dem Betätigungselement durch die verdrehsichere Kopplung drehen kann und somit kann die Drehbewegung von dem Betätigungselement direkt auf das mit dem Kupplungselement verdrehgesicherte Vorschubelement übertragen werden.
Da die Drehbewegung von dem Betätigungselement direkt auf das mit dem Kupplungselement verdrehgesicherte Vorschubelement übertragen wird, kann zum Beispiel eine Übersetzung oder Untersetzung der Axialbewegung realisiert werden, indem zum Beispiel das Gewinde, mit welchem das Betätigungselement zum Beispiel in dem Gehäuse oder einem Gehäuseeinsetzteil der Injektionsvorrichtung geführt wird, eine andere Steigung aufweist als ein Gewinde, insbesondere ein Außengewinde, des Vorschubelements oder der Gewindestange, welche zum Beispiel in einer Gewindeführung oder einem Innengewinde der Injektionsvorrichtung oder des Gehäuses geführt wird. Ist zum Beispiel die Gewindesteigung des Gewindes des Betätigungselements, welches beispielsweise als Außengewinde oder Innengewinde (zum Beispiel eingreifend in ein zum Beispie! gehäusefestes Außengewinde) vorgesehen ist, größer als die Gewindesteigung des Vorschubelements oder der Gewindestange, kann eine Untersetzung bei einem Ausschüttvorgang realisiert werden, das heißt eine zum Beispiel beim Ausschütten von dem Betätigungselement beim Zurückdrehen zurückgelegte axiale Wegstrecke ist größer als eine von dem Vorschubelement oder der Gewindestange zurückgelegte Wegstrecke, so dass ein Benutzer relativ gesehen über eine längere Strecke nur eine kleinere Kraft aufwenden muss, um das Vorschubelement oder die Gewindestange mit einer relativ dazu größeren Kraft über eine relativ dazu kleinere Strecke in Vorschubrichtung vorzubewegen.

Vorzugsweise bewegen sich das Betätigungselement und die Gewindestange zumindest beim Ausschüttvorgang in die gleiche (distale) Richtung. Das Gewinde, in welchem das Betätigungselement geführt wird, ist vorteilhaft nicht selbsthemmend oder selbstsperrend, so dass das Betätigungselement einfach zum Beispiel durch einen in axiale Richtung wirkenden Druck von einem Benutzer in die Injektionsvorrichtung zurück- oder eingeschoben werden kann, wobei sich das Betätigungselement dreht und somit einschraubt, ohne das ein Benutzer bei diesem Ausschüttvorgang eine Drehbewegung vornehmen muss. Der Gewindeeingriff der Gewindestange kann sowohl selbstsperrend oder selbsthemmend als auch nicht-selbstsperrend oder nicht-selbsthemmend sein.
Vorzugsweise kann am Betätigungselement zum Beispiel auf dem distalten Ende des Betätigungselements ein Knopf oder Bedienpunkt vorgesehen sein, welcher beispielsweise drehbar innerhalb des Betätigungselements geführt wird, so dass zum Beispiel ein Benutzer auf den Knopf drücken kann, um eine in axiale Richtung der Injektionsvorrichtung wirkende Kraft auf das Betätigungselement auszuüben, wobei sich beim Einschrauben das Betätigungselement relativ zu dem zum Beispiel durch einen Finger des Benutzers gehaltenen Knopf drehen kann.

Das Kupplungselement kann beispielsweise mittels einer Klauenkupplung mit dem Betätigungselement verdrehsicher gekoppelt und von diesem wieder entkoppelt werden. Dazu sind beispielsweise auf einer ringförmigen oder zumindest zum Teil umlaufenden Fläche sowohl auf dem Betätigungselement als auch auf dem Kupplungselement ein oder mehrere Zacken und/oder Nuten vorgesehen, welche einander gegenüberliegen und welche im ineinander eingreifenden Zustand das Vorschubelement mit dem Kupplungselement verdrehsicher koppeln und welche im nicht-eingreifenden Zustand eine relative Verdrehung zwischen Vorschubelement und Kupplungselement ermöglichen. Die Klauenkupplung ist vorzugweise so ausgestaltet, dass bei Fehlen einer axialen die Kupplung schließenden Kraft die Kupplung geöffnet ist, das heißt das Vorschubelement und das Kupplungselement können relativ zueinander verdreht werden, wenn zum Beispiel ein Benutzer keinen axialen zum Beispiel in distale Richtung gerichteten Druck auf das Kupplungselement direkt oder indirekt, zum Beispiel über einen mit der Kupplung verbundenen oder daran angrenzenden oder in der Nähe der Kupplung liegenden Knopf, ausübt.

Es ist auch möglich, dass ein Kupplungselement zumindest bei einer Ausschüttbewegung oder dauerhaft mit dem Betätigungselement verdrehgesichert ist und nur während oder vor einem Ausschütt-Vorgang verdrehsicher mit dem Vorschubelement gekoppelt wird.

Das Kupplungselement kann einteilig oder auch aus mehreren Teilen oder Kupplungen zusammengesetzt werden, solange eine Entkopplung des Betätigungselementes vom Vorschubelement und vorzugsweise eine relative Verdrehbarkeit dieser Elemente während eines Einstellvorganges realisiert werden kann und eine verdrehsichere Kopplung dieser Elemente vor und/oder während eines Abgabe- oder Ausschüttvorganges realisiert werden kann.

Es ist eine Rückdrehsicherung zum Beispiel in Form eines bekannten Ratschen-Mechanismus in der Dosiervorrichtung vorgesehen, mit welcher die Gewindestange einseitig relativ zur Dosiervorrichtung verdrehgesichert werden kann, d.h. die Gewindestange kann sich relativ zur Dosiervorrichtung nur in einer (Vorschub-)Richtung drehen, wohingegen eine Drehung in Gegenrichtung durch die Rückdrehsicherung verhindert wird, so dass zum Beispiel nur ein in distale Richtung gerichtetes Einschrauben der in der Dosiervorrichtung
gewindegeführten Gewindestange in die Injektionsvorrichtung möglich ist.
Die Gewindestange kann in einem unidirektionalen Verdrehsicherungselement, wie zum Beispiel einem Ratschenkupplungsteil, geführt werden. Ein Rückdrehsicherungselement kann zum Beispiel verdrehsicher mit der Gewindestange gekoppelt sein, indem zum Beispiel am Rückdrehelement ein oder mehrere zum Beispiel in radiale Richtung auf das Vorschubelement hin gerichtete Vorsprünge oder Stege vorgesehen sind, welche zum Beispiel in axiale Längsnuten oder Längsrillen der Gewindestange eingreifen können. Ebenso kann eine verdrehsichere Kupplung auch dadurch erfolgen, dass Vorsprünge der Gewindestange in korrespondierende Ausnehmungen der Rückdrehsicherung oder des Rückdrehsperrelements eingreifen. Das Rückdrehsperrelement kann zum Beispiel ein oder mehrere radial nach außen vorgespannte Elemente oder Arme aufweisen, welche beispielsweise mit auf der Innenseite der Injektionsvorrichtung oder eines Gehäuses vorgesehenen Stufen oder umlaufenden Vorsprüngen so zusammenwirken, dass diese radial nach außen vorgespannten Arme die zum Beispiel innenseitig umlaufenden Stufen in eine Richtung überfahren können und nach Überfahren einer Stufe einschnappen, also eine Zurückdrehung blockieren.

Das Sperrelement ist mit einer Einheit oder einem Element zum festlegen einer maximal einstellbaren Dosis gekoppelt, zum Beispiel darauf aufgeschnappt. Das Sperrelement ist mit einem solchen Stoppelement axial verschiebesicher gekoppelt, so dass das Stoppelement das Sperrelement bei einer Bewegung in axialer Richtung mitnimmt, wobei eine relative Drehung möglich ist. Bei dieser relativen Drehung werden Klick-Geräusche erzeugt, indem beispielsweise elastische oder Federarme der Rückdrehsicherung oder des Sperrelements in eine korrespondierende Zahnung des Stoppelements oder der Rückdrehsicherung eingreifen.
Vorteilhaft ist ein Auslöseknopf zum Beispiel am Kupplungselement und/oder am Betätigungselement vorgesehen und vorteilhaft ist der Auslöseknopf drehbar gelagert, wobei sich der Auslöseknopf zum Beispiel relativ zum Kupplungselement um dessen Längsachse drehen kann. Dabei ist der Auslöseknopf vorteilhaft in der Nähe der oben erwähnten Kupplung, also zum Beispiel im proximalen Bereich der Injektionsvorrichtung, vorgesehen, so dass ein Druck auf den Auslöseknopf das Einkuppeln des Kupplungselementes bewirkt und so zum Beispiel eine Verdrehsicherung zwischen dem Betätigungselement und dem Vorschubelement oder der Gewindestange herstellt, wobei bei fortgesetztem Druck auf den Auslöseknopf das Betätigungselement zusammen mit der Gewindestange in die Injektionsvorrichtung in distale Richtung eingeschoben wird und eine Relativdrehung zwischen dem drehbar gelagerten Auslöseknopf einerseits und dem Betätigungselement und dem Vorschubelement andererseits stattfindet.

Bei einem Verfahren zum Einstellen und Abgeben einer Dosis aus einer Injektionsvorrichtung wird oder ist ein Betätigungs- und/oder Einstellelement beim Einstellen einer Dosis von einem Vorschubelement zumindest bezüglich der Übertragung einer Drehbewegung entkoppelt, so dass eine Dosis durch ein Drehen des Betätigungselementes eingestellt und optional auch durch Zurückdrehen korrigiert werden kann. Vor oder während der Abgabe einer Dosis wird das Betätigungselement mit dem Vorschubelement zum Beispiel mittels eines Kupplungselementes oder einer Kupplung verdrehsicher gekoppelt, so dass eine Drehbewegung des zum Beispiel in einer Dosiervorrichtung oder Injektionsvorrichtung gewindegeführten Betätigungselementes unmittelbar als Drehbewegung auf das Vorschubelement oder eine Gewindestange übertragen werden kann. Dabei tritt zum Beispiel keine Ober- oder Untersetzung der Drehung des Betätigungselementes auf, so dass sich vorteilhaft das Betätigungselement und das Vorschubelement durch die verdrehsichere Kopplung immer um den gleichen Winkel zusammen drehen.

Gemäß der Erfindung weist die Dosiervorrichtung ein Begrenzungselement auf, welches mit einem drehbaren und/oder verschiebbaren Einstellelement so gekoppelt und zum Beispiel verdrehgesichert ist, dass das Begrenzungselement bei einer Bewegung des Einstellelementes in Richtung auf eine Begrenzung oder einen Anschlag bewegt wird und wobei das Begrenzungselement relativ zur Dosiervorrichtung oder Injektionsvorrichtung wieder in die Ausgangsstellung zurückbewegt wird, wenn die eingestellte Dosis zum Beispiel durch Zurückbewegen des Einstellelementes abgegeben wird.

Vorteilhaft bewegt sich somit das Begrenzungselement nur zwischen zwei Positionen und führt somit eine Art "Pendelbewegung" aus. Dabei wird bevorzugt der Anschlag oder die Begrenzung beim Einstellen der Dosis und/oder vorzugsweise nur beim Abgeben der Dosis in Richtung auf das Begrenzungselement zu bewegt, so dass die Distanz oder der Abstand zwischen dem Begrenzungselement und dem die eigentliche Begrenzung oder Blockierung verursachenden Anschlag oder der Begrenzung, welche beispielsweise auf einer sich axial verschiebenden Gewindestange vorgesehen ist, bei jeder Dosisabgabe verkürzt wird, so dass die nach der erfolgten Dosisabgabe durchzuführende maximale einzustellende Gesamtdosierung verringert wird.

Die Erfindung wird nachfolgend anhand von Ausführungsbeispielen beschrieben.

Es zeigen:
- Figur 1: eine Querschnittsansicht der Injektionsvorrichtung gemäß einer ersten Ausführungsform;
- Figur 2: eine Detailansicht der in Figur 1 gezeigten Dosier- und Ausschüttmechanik;
- Figur 3: eine Explosionszeichnung der in Figur 1 gezeigten Injektionsvorrichtung;
- Figur 4: das Gehäuse der Injektionsvorrichtung;
- Figur 5: eine Gewindehülse der Injektionsvorrichtung;
- Figur 6: eine Dosierhülse der Injektionsvorrichtung;
- Figur 7: ein Kupplungselement der Injektionsvorrichtung;
- Figur 8: ein Dosierknopf der Injektionsvorrichtung;
- Figur 9: eine Gewindestange der injektionsvorrichtung
- Figur 10: eine Stopphülse oder Stoppmutter der Injektionsvorrichtung;
- Figur 11: eine Rückdrehsicherung der Injektionsvorrichtung;

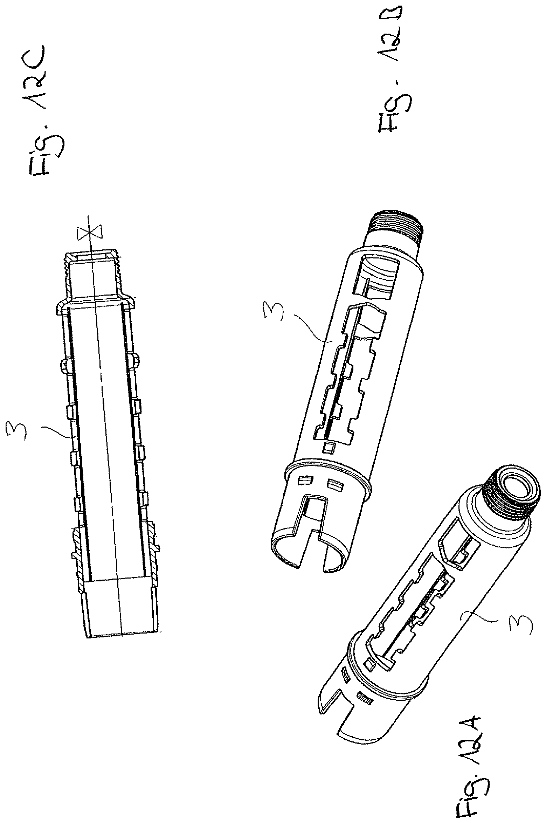

- Figur 12: eine Karpulen - oder Ampullenhülse der Injektionsvorrichtung;

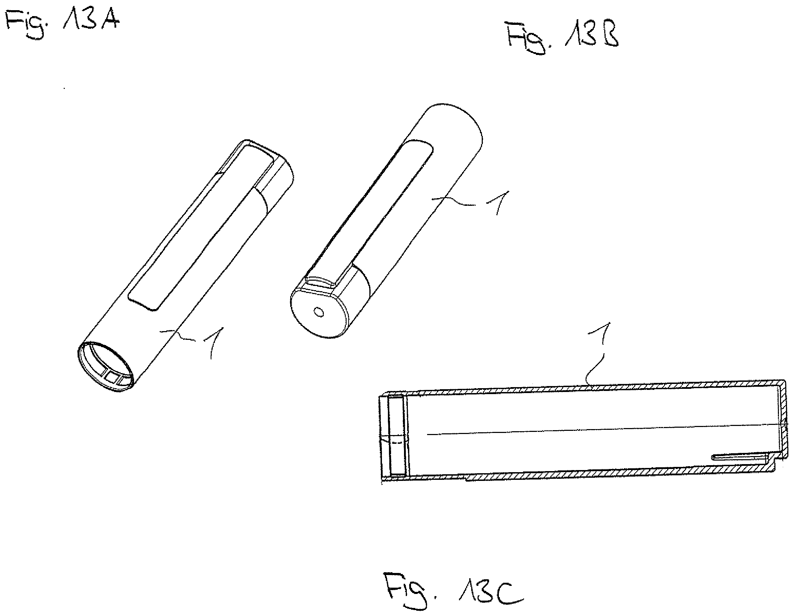

- Figur 13: eine Schutzkappe der Injektionsvorrichtung;
- Figur 14A: eine Querschnittsansicht einer weiteren Ausführungsform einer Injektionsvorrichtung im Auslieferungszustand;
- Figur 14B: die in Figur 14A gezeigte Injektionsvorrichtung nach Abgeben einer Dosis im aufgezogenen Zustand;
- Figur 14C: die Kopplung zwischen Einstellelement und Stopp-Element;
- Figur 14D: die Führung einer Gewindestange in einem Kupplungselement;
- Figur 14E: eine perspektivische Ansicht des Einstell- oder Betätigungselementes;
- Figur 14 F: eine Kopplung der Rückdrehsicherung mit dem Pendelstopp.

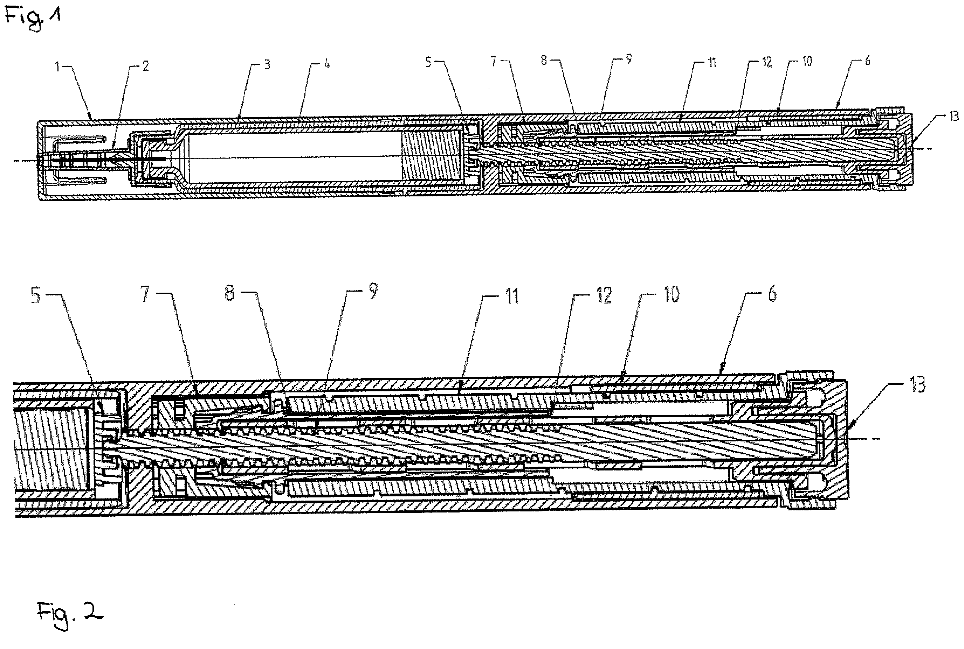

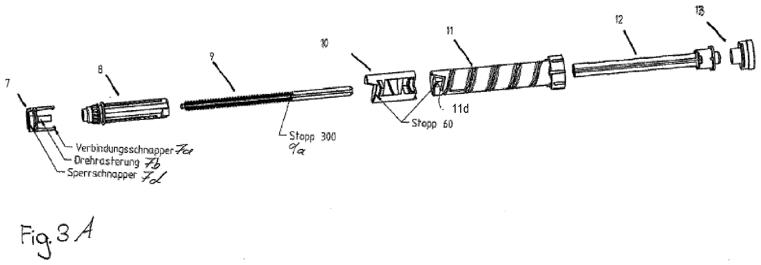

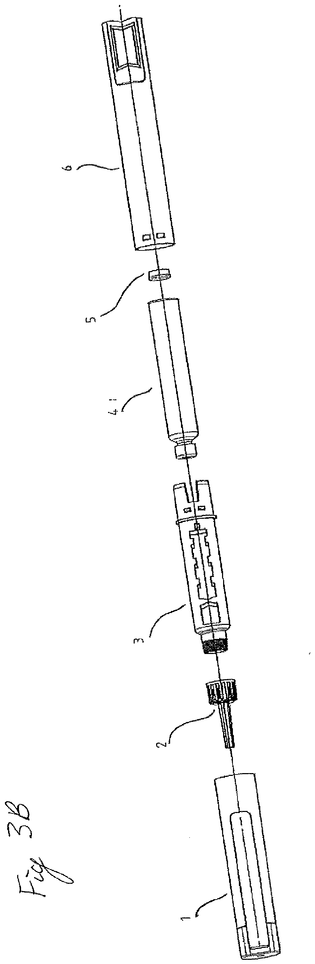

Der Pen besteht, wie in den Figuren 1 bis 3 gezeigt, aus einer Dosiermechanik, einem Flansch 5, einer Karpule oder Ampulle 4 mit Medikament, einem Karpulenhalter 3, einer Nadel 2 und einer Schutzkappe 1.

Der Flansch 5 wird auf die Gewindestange 9, die aus der Dosiermechanik herausschaut, aufgeschnappt. Die Karpule 4 wird zwischen Karpulenhalter 3 und Gehäuse 6 der Dosiermechanik, die ineinander verschnappt sind, festgehalten. Die Dosiermechanik besteht aus der Kraftübersetzung, den Stoppelementen und den Kupplungselementen.

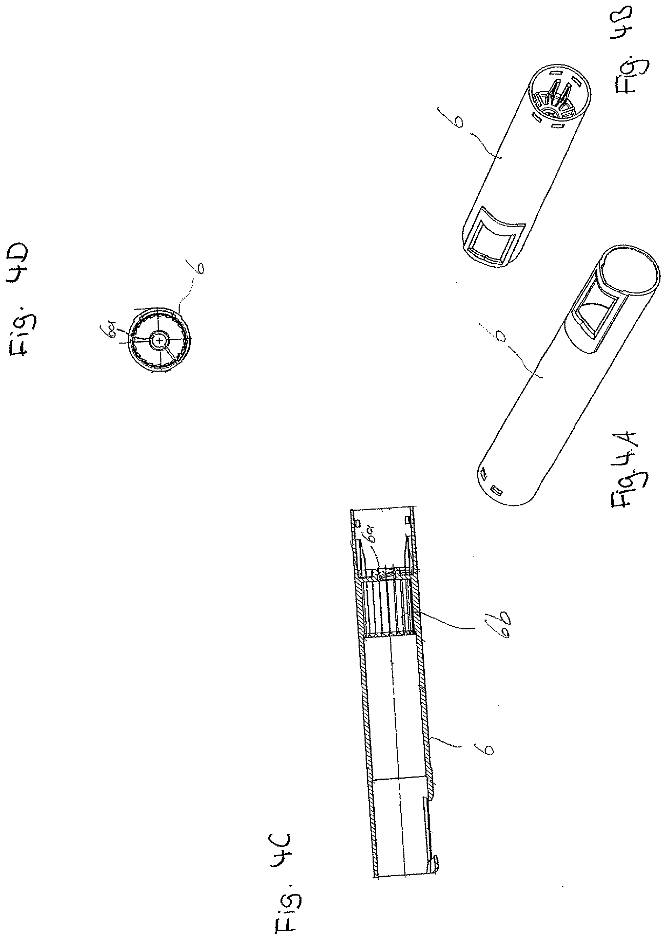

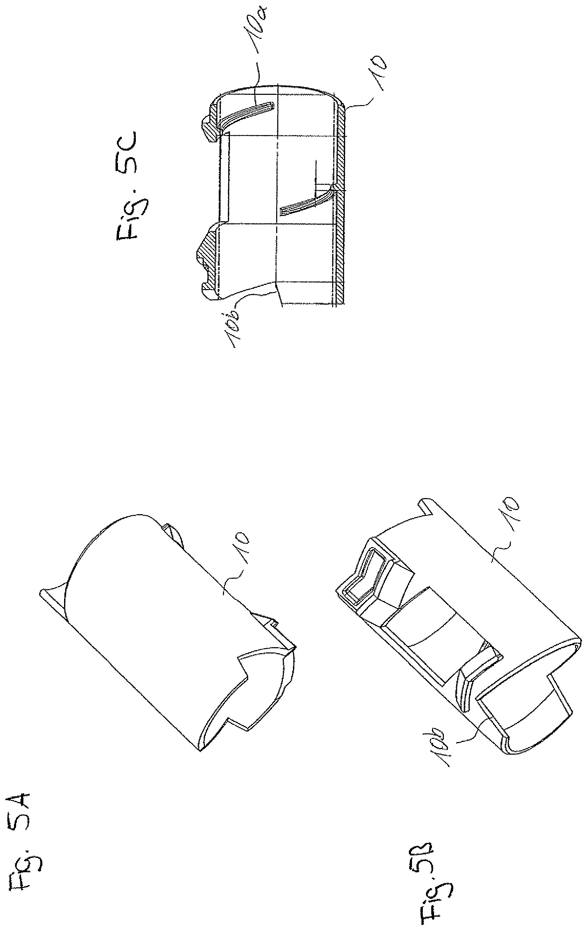

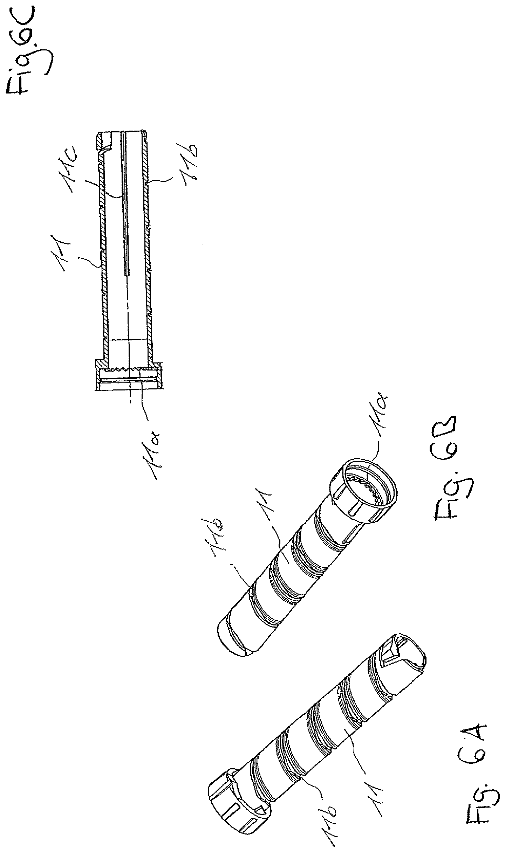

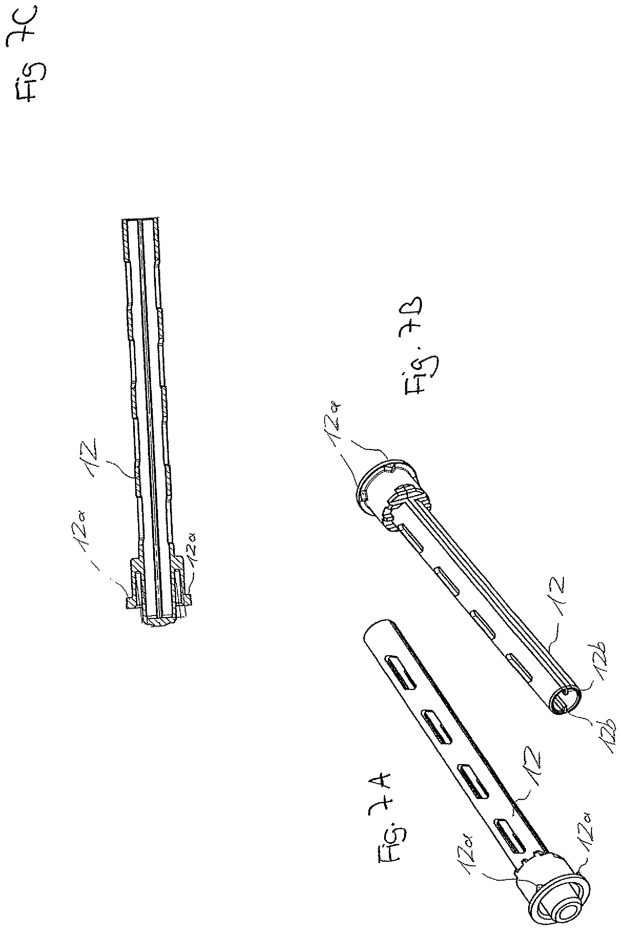

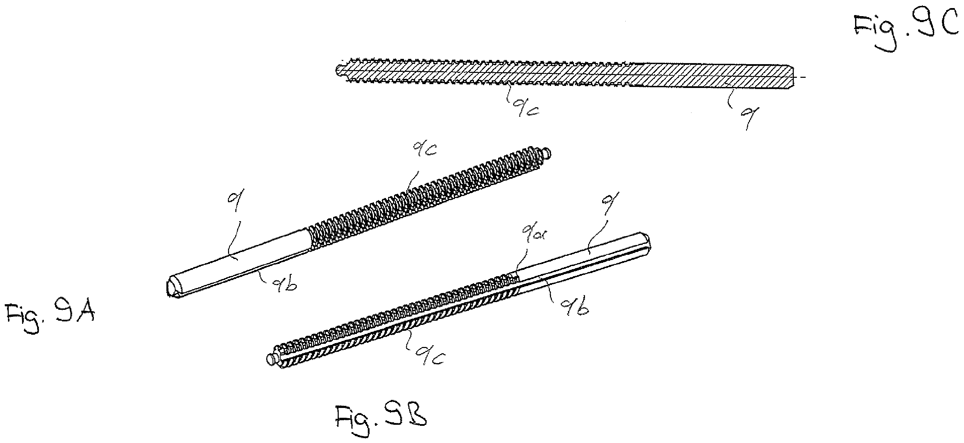

Die Kraftübersetzung besteht aus einer im Gehäuse 6 (Figur 4) arretierten Gewindehülse 10 (Figur 5), in deren Gewinde 10a ein herausdrehbares Betätigungselement 11 (Figur 6) läuft, das über die Kupplung 12 (Figur 7) eine Gewindestange 9 (Figur 9) dreht, die im Gewinde des Gehäuses 6 läuft.

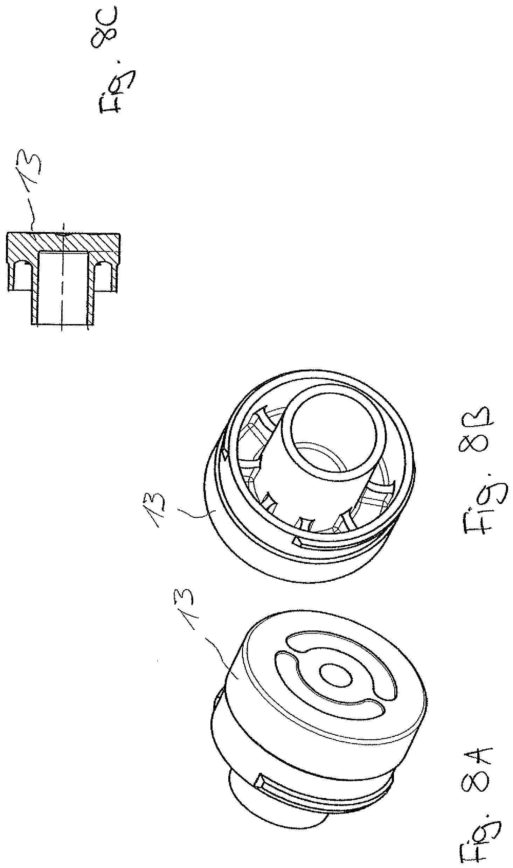

Die Kupplungselemente bestehen aus einer auf der Gewindestange 9 linear geführten Kupplung 12, deren Zähne 12a im Eingriff mit den Zähnen 11a des Betätigungselementes 11 sein können, und dem Dosierknopf 13 (Figur 8), der die Eingangskraft des Benutzers aufnimmt und an die Kupplung 12 weiterleitet.

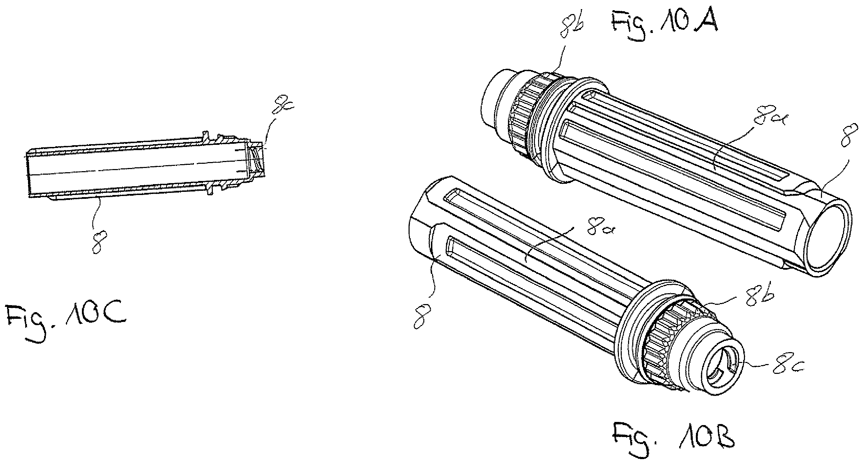

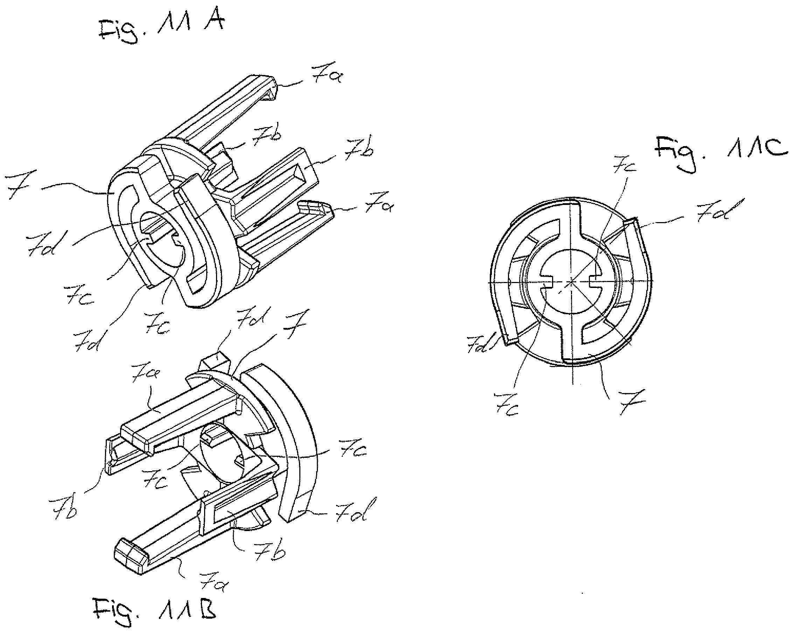

Die Stoppelemente bestehen aus einer Stopphülse 8 (Figur 10) die mit dem Gewindeende 9a der Gewindestange 9 die Maximaldosisbegrenzung bzw. den Stopp 300 bildet, und einer Rückdrehsicherung 7 (Figur 11), die die Verdrehung der Gewindestange 9 zum Gehäuse 6 in eine Richtung verhindert. Sie ist axial mit der Stopphülse 8 mittels der Rastarme 7a verschnappt und bildet mit ihr einen radialen Dosierklick mittels der Arme 7b.

Figur 1 zeigt eine Dosiervorrichtung einer Injektionsvorrichtung, bei welcher die Dosierfunktion, also die Einschubbewegung des beim Aufdosieren herausgedrehten Betätigungselements 11, durch eine Drehbewegung der nach dem Aufdosieren eingekoppelten Kupplung 12 direkt auf die Gewindestange 9 übertragen wird.

Es handelt sich hierbei um einen Dreh-Pen, das heißt die axiale Bewegung wird komplett in eine Drehbewegung umgesetzt und wieder zurücktransformiert. Das Betätigungselement 11 drückt nicht unmittelbar auf die Gewindestange 9.

Die Gewindestange 9 ist über eine unidirektionale Ratschenkupplung 7 mit dem Gehäuse 6 drehgesichert. Die Ratschenkupplung 7 ist über eine Keil-Nut-Verbindung 7c, 9b zur Gewindestange 9 drehgesichert. Die Gewindestange 9 kann sich folglich nur nach vorne in die distale Richtung geführt durch das Gewinde 6a des Gehäuses 6 schrauben.

Das Betätigungselement 11 ist mittels eines Außengewindes 11b in einem Innengewinde 10a der gehäusefesten Gewindehülse 10 oder des Gehäuses 6 geführt. Beim Aufdosieren wird das Betätigungselement 11 aus dem Gehäuse 6 herausgedreht. Das Betätigungselement 11 bewegt sich dabei axial und in Drehrichtung relativ zur im Gehäuse 6 gehaltenen Gewindestange 9.

Eine Stoppmutter 8 ist durch eine in axialer Richtung verlaufenden Nut 8a, in die ein axial verlaufender Steg 11c des Betätigungselementes 11 eingreift, relativ zu dem Einstell- oder Betätigungselement 11 verdrehgesichert, aber relativ dazu axial verschiebbar.

Bei einer Drehbewegung des Betätigungselementes 11 wird die mit dem Betätigungselement 11 verdrehgesicherte Stoppmutter 8 mitgedreht. Die Gewindestange 9 ist im Gehäuse 6 beim Aufdosieren oder Herausdrehen des Betätigungselementes 11 durch die Ratschenkupplung 7 verdrehgesichert. Dadurch schraubt sich die Stoppmutter 8 auf der Gewindestange 9 in proximale Richtung.

Vor Beginn der Ausschüttbewegung wird die Gewindestange 9 verdrehsicher mit dem Betätigungselement 11 gekoppelt. Axial sind Betätigungselement 11 und Gewindestange 9 jedoch relativ zueinander bewegbar. Als Kupplung 12 ist ein außerhalb der Gewindestange 9 geführtes Element vorgesehen, welches mit der Gewindestange 9 durch eine Keil-Nut 9b, 12b verdrehgesichert ist. Stirnseitig sind an dem Betätigungselement 11 in proximale Richtung weisend umlaufend Kronen 11a vorgesehen, welche in stirnseitig vorgesehene in distale Richtung weisende gegenüberliegende Klauen 12a des Kupplungselementes 12 eingreifen können. Beim Herausdrehen des Betätigungselementes 11 kann sich das Betätigungselement 11 relativ zum Kupplungselement 12 drehen, da keine Kraft in distale Richtung auf das Kupplungselement 12 wirkt. Die Klauen 12a und Kronen 11a überrasten bzw. rutschen bei der Drehbewegung des Betätigungselementes 11 aneinander vorbei. Beim Aufdosieren ist das Kupplungselement 12 verdrehgesichert im Gehäuse 6 aufgrund der Verdrehsicherung der Gewindestange 9, welche relativ zum Gehäuse 6 durch eine unilaterale Ratschenkupplung 7 verdrehgesichert ist.

Beim Auslösen wird über den auf das Kupplungselement 12 aufgeschnappten Druckknopf 13 auf das Kupplungselement 12 in distale Richtung gedrückt, so dass die Kupplung 12 mit dem Betätigungselement 11 verdrehgesichert wird aufgrund des Eingriffs der ineinander greifenden Kronen 11a und Klauen 12a. Das Betätigungselement 11 dreht sich beim Einschieben aufgrund der Gewindeführung 11b, 10a im Innengewinde innerhalb des Gehäuses 6. Durch die Verdrehsicherung des Betätigungselementes 11 mit dem Kupplungselement 12 wird die Drehbewegung des Betätigungselementes 11 auf die mit der Kupplung 12 verdrehgesicherte Gewindestange 9 übertragen. Die Gewindestange 9 dreht sich somit und wird, geführt durch das Innengewinde 6a des Gehäuses 6, in distale Richtung eingeschraubt.

Die Stoppmutter 8 läuft auf der Gewindestange 9 und hat ein Innengewinde 8c, in welches das Außengewinde 9c der Gewindestange 9 eingreift.

Die Stoppmutter 8 schraubt sich relativ zur Gewindestange 9 nur beim Aufdosieren in proximale Richtung. Beim Ausschütten bleibt die Stoppmutter 8 relativ zur Gewindestange 9 in der gleichen Position, da das Betätigungselement 11 und die Gewindestange 9 durch das Kupplungselement 12 verdrehgesichert sind, und wird zusammen mit der Gewindestange 9 in ihre Ausgangslage zurückgefahren. Somit ist die Stoppmutter 8 im Pen nach Aufzieh- und Ausschüttbewegung wieder and der gleichen Position (Pendelbewegung). Die Position auf der Gewindestange 9 hat sich jedoch beim Aufziehen verändert. Die Stoppmutter 8 ist relativ zur Gewindestange 9 beim Aufziehen in proximale Richtung gefahren. Die Stopp-Funktion der Stoppmutter 8 ergibt sich durch einen Anschlag 9a auf der Gewindestange 9, welcher verhindert, dass das Betätigungselement 11 beim Aufdosieren weiter herausgedreht werden kann, wenn die Stoppmutter 8 am Anschlag anliegt.

Nur die Gewindestange 9 durchfährt einen vorgegebenen maximalen durch die Anfangs-Axialposition der Stoppmutter 8 an der Gewindestange 9, vorzugsweise den Axialabstand von Anfang-Axialposition der Stoppmutter 8 zu einem Gewindestangenanschlag, definierten Weg, der der total abzugebenden Medikamentenmenge der Ampulle von zum Beispiel 300 Einheiten entspricht, nicht jedoch die Stoppmutter 8, welche lediglich eine Pendelbewegung innerhalb des Pens ausführt.

Durch diese Anordnung der Stoppmutter 8 kann die Stopp-Funktion schon beim Aufdosieren gewährleistet werden, da im Falle der letzten abzugebenden Dosis die Stoppmutter 8 bereits an dem Anschlag 9a der Gewindestange 9 anschlägt und ein weiteres Aufdosieren oder Herausdrehen des Betätigungselementes 11 blockiert.

Die Gewindestange 9 weist relativ zum Gehäuse 6 eine Rückdrehsicherung in Form einer unidirektionalen Kupplung 7 auf. Die Rückdrehsicherung 7 ist axial mit einem Schnapphaken 7a mit dem Pendelstopp 8 gekoppelt.

Pendelstopp 8 und Rückdrehsicherung 7 sind über einen Dosierklick, erzeugt durch die Arme 7b, die die Zähne 8b der Stoppmutter 8 überfahren, relativ zueinander drehbar.

Da alle Funktionen wie Dosisbegrenzung und Rückdrehsicherung innerhalb der Injektionsvorrichtung integriert sind, kann das Einstellelement beziehungsweise der Dosierknopf des Betätigungselements 11, bzw. der im Kupplungselement 8 gehaltene Auslöseknopf 13, relativ flach gehalten werden, das heisst die Bauhöhe des Dosierknopfes kann abgeflacht werden, wodurch die Hubbewegung für den Daumen eines Patienten zum Injizieren der eingestellten Dosis verringert werden kann.

Beim Ausschütten sind Pendelstopp 8 und Rückdrehsicherung 7 gekoppelt, also drehfest, so dass sich keine Klick-Geräusche ergeben.

Die Rückdrehsicherung 7 hat radial nach außen vorgespannte Federarme 7d, welche in Rasterungen 6b des umlaufenden Gehäuses 6 eingreifen. Beim Ausschütten dreht sich die Rückdrehsicherung 7 relativ zum Gehäuse 6 und kann somit Klick-Geräusche erzeugen. Bei der Dosiseinstellung oder Dosiskorrektur ist die Rückdrehsicherung 7 gehäusefest und erzeugt somit keine Klick-Geräusche.

Beim Klicken kann außer der Erzeugung eines Geräusches auch ein taktiles Feedback für einen Benutzer gegeben werden.

Ein Anschlag 11d des Betätigungselements 11 und ein Anschlag 10b der Gewindehülse 10 bilden eine Begrenzung für die maximal einstellbare Einzeldosis (sogenannter Stopp 60).

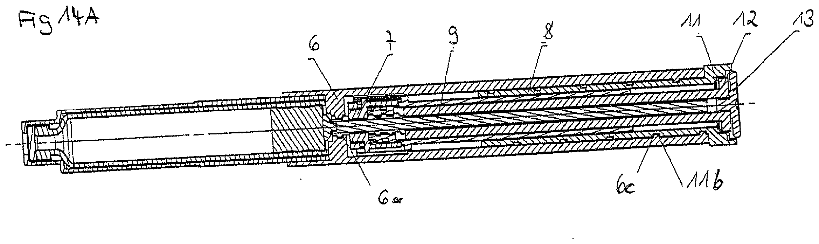

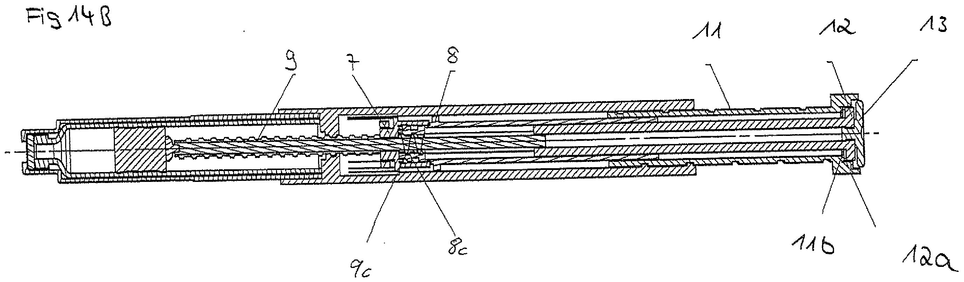

Figur 14A zeigt eine Dosiervorrichtung einer Injektionsvorrichtung gemäß einer zweiten Ausführungsform, bei welcher die Dosierfunktion, also zum Beispiel die Einschubbewegung des beim Aufdosieren herausgedrehten Betätigungselements 11 (siehe Fig. 14B), durch eine Drehbewegung der nach dem Aufdosieren eingekoppelten Kupplung 12 direkt auf die Gewindestange 9 übertragen wird.

Zum Beispiel kann eine Übersetzung und/oder Untersetzung zum Beispiel zwischen einem Einstell- oder Dosierelement 11 und einem Vorschubelement 9 insbesondere beim Aufdosieren und/oder bei der Dosisabgabe realisiert werden.

Es handelt sich hierbei um einen Dreh-Pen, das heißt die axiale Bewegung wird komplett in eine Drehbewegung umgesetzt und wieder zurücktransformiert. Das Betätigungselement 11 drückt nicht unmittelbar auf die Gewindestange 9.

Die Gewindestange 9 ist rückdrehgesichert, zum Beispiel durch eine Ratschenkupplung 7. Die Ratschenkupplung 7 ist zum Beispiel am Gehäuse 6 angebracht. Die Ratschenkupplung 7 ist zum Beispiel mit der Gewindestange 9 durch eine Keil-Nut-Verbindung drehgesichert. Als weitere Ausführungsform kann die Ratschenkupplung 7 auch mit einem zusätzlichen, gegenläufigen Gewinde auf der Gewindestange 9 umgesetzt werden. Die Gewindestange 9 kann sich folglich nur nach vorne in distale Richtung, geführt durch das Gewinde 6a des Gehäuses 6, schrauben.

Das Betätigungselement 11 ist mittels eines Außengewindes 11b in einem Innengewinde 6c des Gehäuses 6 geführt. Beim Aufdosieren wird das Betätigungselement 11 aus dem Gehäuse 6 herausgedreht, wie in Fig. 14B gezeigt. Das Betätigungselement 11 bewegt sich dabei axial und in Drehrichtung relativ zur im Gehäuse 6 gehaltenen Gewindestange 9.

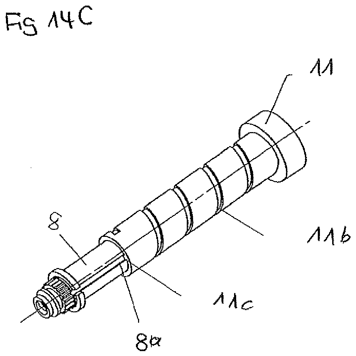

Eine Stoppmutter 8 ist verdrehgesichert relativ zu dem Einstell- oder Betätigungselement 11, zum Beispiel durch einen in axiale Richtung verlaufenden in Figur 14C gezeigten Steg oder Vorsprung 11c auf der Innenseite des Betätigungselements 11, welcher in eine axial verlaufende Nut 8a der Stoppmutter 8 eingreift. Die Stoppmutter 8 läuft auf der Gewindestange 9 und hat ein Innengewinde 8c, in welches das Außengewinde 9c der Gewindestange 9 eingreift.

Bei einer Drehbewegung des Betätigungselementes 11 wird die mit dem Betätigungselement 11 verdrehgesicherte Stoppmutter 8 mitgedreht. Die Gewindestange 9 ist im Gehäuse 6 beim Aufdosieren oder Herausdrehen des Betätigungselementes 11 verdrehgesichert. Dadurch schraubt sich die Stoppmutter 8 auf der Gewindestange9 in proximale Richtung.

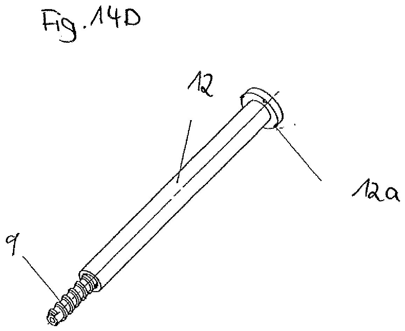

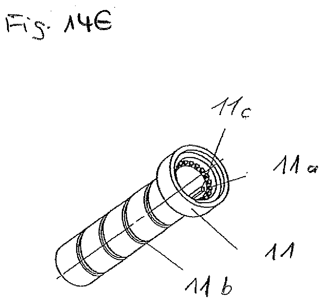

Vor Beginn der Ausschüttbewegung wird die Gewindestange 9 verdrehsicher mit dem Betätigungselement 11 gekoppelt. Axial sind Betätigungselement 11 und Gewindestange 9 jedoch relativ zueinander bewegbar. Als Kupplung 12 ist ein außerhalb der Gewindestange 9 geführtes Element vorgesehen, wie in Figur 14D gezeigt, welches mit der Gewindestange 9 zum Beispiel durch Formschluß verdrehgesichert ist. Stirnseitig sind an dem Betätigungselement 11 in proximale Richtung weisend umlaufend Kronen oder Klauen 11a vorgesehen, wie in Figur 14E gezeigt, welche in stirnseitig vorgesehene in distale Richtung weisende gegenüberliegende umlaufende Kronen oder Klauen 12a des Kupplungselementes 12 eingreifen können. Beim Herausdrehen des Betätigungselementes 11 kann sich das Betätigungselement 11 relativ zum Kupplungselement 12 drehen, da keine Kraft in distale Richtung auf das Kupplungselement 12 wirkt. Die Klauen 11a und 12a überrasten bzw. rutschen bei der Drehbewegung des Betätigungselementes 11 aneinander vorbei. Beim Aufdosieren ist das Kupplungselement 12 verdrehgesichert im Gehäuse 6 aufgrund der Verdrehsicherung innerhalb der Gewindestange 9, welche relativ zum Gehäuse 6 zum Beispiel durch einen Ratschen-Mechanismus verdrehgesichert ist.

Beim Auslösen wird auf die Kupplung oder das Kupplungselement 12 in distale Richtung, zum Beispiel durch Druck auf einen Auslöseknopf 13, gedrückt, so dass die Kupplung oder das Kupplungselement 12 mit dem Betätigungselement 11 verdrehgesichert wird aufgrund des Eingriffs der ineinander greifenden Kronen oder Klauen 11a und 12a. Das Betätigungselement 11 dreht sich beim Einschieben aufgrund der Gewindeführung im Innengewinde 6c innerhalb des Gehäuses 6. Durch die Verdrehsicherung des Betätigungselementes 11 mit der Kupplung oder dem Kupplungselement 12 wird die Drehbewegung des Betätigungselementes 11 auf die mit der Kupplung oder dem Kupplungselement 12 verdrehgesicherte Gewindestange 9 übertragen. Die Gewindestange 9 dreht sich somit und wird, geführt durch das Innengewinde 6a des Gehäuses 6, in distale Richtung eingeschraubt.

Die Stoppmutter 8 schraubt sich relativ zur Gewindestange 9 nur beim Aufdosieren in proximale Richtung. Beim Ausschütten bleibt die Stoppmutter 8 relativ zur Gewindestange 9 in der gleichen Position, da das Betätigungselement 11 und die Gewindestange 9 durch das Kupplungselement 12 verdrehgesichert sind, und wird zusammen mit der Gewindestange 9 in ihre Ausgangslage zurückgefahren. Somit ist die Stoppmutter 8 im Pen nach Aufzieh- und Ausschüttbewegung wieder an der gleichen Position (Pendelbewegung). Die Position auf der Gewindestange 9 hat sich jedoch beim Aufziehen verändert. Die Stoppmutter 8 ist relativ zur Gewindestange 9 beim Aufziehen in proximale Richtung gefahren. Die Stopp-Funktion der Stoppmutter 8 ergibt sich zum Beispiel durch einen Anschlag (axial oder in Umfangsrichtung) auf der Gewindestange 9, welcher verhindert, dass das Betätigungselement 11 beim Aufdosieren weiter herausgedreht werden kann, wenn die Stoppmutter 8 am Anschlag anliegt.

Figur 14B zeigt einen solchen Zustand, wobei die Stoppmutter 8 das Gewindeende der Gewindestange 9 erreicht hat und somit nicht mehr weitergedreht werden kann, so dass ein weiteres Aufdosieren oder Herausdrehen des Betätigungselements 11 nicht möglich ist.

Bei dieser Ausführungsform durchfährt nur die Gewindestange 9 einen vorgegebenen maximalen durch die Anfangs-Axialposition der Stoppmutter 8 auf der Gewindestange 9, vorzugsweise den Axialabstand von Anfangs-Axialposition der Stoppmutter 8 zu einem Gewindestangenanschlag, definierten Weg, der zum Beispiel der total abzugebenden Medikamentenmenge der Ampulle, wie zum Beispiel abzugebenden 300 Einheiten, entspricht, nicht jedoch die Stoppmutter 8, welche lediglich eine Pendelbewegung innerhalb des Pens ausführt.

Durch diese Anordnung der Stoppmutter 8 kann die Stopp-Funktion schon beim Aufdosieren gewährleistet werden, da im Falle der letzten abzugebenden Dosis die Stoppmutter 8 bereits an dem Anschlag der Gewindestange 9 anschlägt und ein weiteres Aufdosieren oder Herausdrehen des Betätigungselementes 11 blockiert.

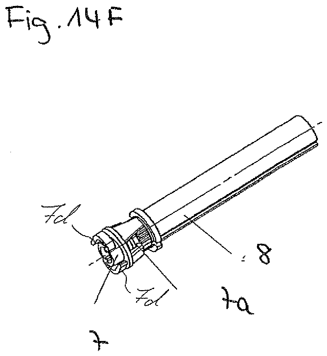

Die Gewindestange 9 weist relativ zum Gehäuse 6 eine Rückdrehsicherung zum Beispiel in Form einer an sich bekannten uni-direktionalen Kupplung 7 auf. Die Rückdrehsicherung ist in Figur 14F axial mit dem Pendelstopp 8 gekoppelt, zum Beispiel mit einem Schnapphaken 7a. Pendelstopp 8 und Rückdrehsicherung 7 sind relativ zueinander frei drehbar.

Da alle Funktionen, wie zum Beispiel Dosisbegrenzung und Rückdrehsicherung, innerhalb der Injektionsvorrichtung integriert sind, kann das Einstellelement beziehungsweise der Dosierknopf, also zum Beispiel der proximale Teil des Betätigungselements 11 und auch der zum Beispiel im Kupplungselement 12 gehaltene Auslöseknopf 7, relativ flach gehalten werden, das heißt die Bauhöhe des Dosierknopfes kann abgeflacht werden, wodurch die Hubbewegung für den Daumen eines Patienten zum Injizieren der eingestellten Dosis verringert werden kann.

Bei einem Ausführungsbeispiel kann zwischen dem Pendelstopp 8 und der Rückdrehsicherung 7 ein Klick-Element angeordnet werden, so dass zum Beispiel ein vom Pendelstopp 8 radial nach außen vorgespannter Federarm in Rastelemente der umlaufenden Rückdrehsicherung 7 eingreift oder umgekehrt.

Beim Ausschütten sind Pendelstopp 8 und Rückdrehsicherung 7 gekoppelt, also drehfest, so dass sich keine Klick-Geräusche ergeben.

Die Rückdrehsicherung 7 hat zum Beispiel radial nach außen vorgespannte Federarme 7d , welche in Rasterungen des umlaufenden Gehäuses 6 eingreifen.

Beim Ausschütten dreht sich die Rückdrehsicherung 7 relativ zum Gehäuse 6 und kann somit Klick-Geräusche erzeugen. Bei der Dosiseinstellung oder Dosiskorrektur ist die Rückdrehsicherung 7 gehäusefest und erzeugt somit keine Klick-Geräusche.
Beim Klicken kann außer der Erzeugung eines Geräusches auch ein taktiles Feedback für einen Benutzer gegeben werden.
Bei einer alternativen Ausführungsform ist der Klick-Mechanismus nicht zwischen Pendelstopp 8 und Rückdrehsicherung 7 angeordnet, sondern Pendelstopp 8 und Rückdrehsicherung 7 weisen jeweils Klick-Elemente auf, welche zum Beispiel radial nach außen vorgespannte Federarme sind und in Rasterungen des Gehäuses 6 eingreifen. Hierdurch können so genannte an sich nicht erwünschte "Doppel-Klicks" beim Ausschütten erzeugt werden, da sich hierbei sowohl Pendelstopp 8 als auch Rückdrehsicherung 7 relativ zum Gehäuse drehen. Beim Dosiseinstellen wird nur ein "Einfach-Klick" durch den Pendelstopp 8 erzeugt, da die Rückdrehsicherung 7 gehäusefest ist.

## Patentansprüche

1. Dosiervorrichtung für eine Injektionsvorrichtung
mit einem Betätigungs- oder Einstellelement (11) zum Einstellen und Abgeben einer Dosis aus einer Injektionsvorrichtung,
mit einem Vorschubelement (9) zum Erzeugen einer Vorschubbewegung zum Ausschütten einer Dosis,
mit einer Kupplung (12), mit welcher das Betätigungs- oder Einstellelement (11) und das Vorschubelement (9) zum Abgeben der mit dem Betätigungs- oder Einstellelement (11) eingestellten Dosis so gekoppelt werden können, dass eine Drehbewegung oder Ausschüttbewegung des Betätigungs- oder Einstellelement (11) direkt auf das Vorschubelement (9) übertragen wird,
mit einem Begrenzungselement (8) zum Begrenzen einer bei einer Injektionsvorrichtung maximal einstellbaren Gesamtdosis, wobei das Betätigungs- oder Einstellelement (11) so mit dem Begrenzungselement (8) gekoppelt ist, dass sich das Begrenzungselement (8) bei einer Bewegung des Betätigungs- oder Einstellelementes (11) von einer Ausgangsposition innerhalb der Dosiervorrichtung in Richtung auf eine Begrenzung oder einen Anschlag zum Sperren der Bewegung des Begrenzungselementes (8) bewegt, um dadurch die Bewegung des Betätigungs- oder Einstellelementes (11) in eine Einstellrichtung zu blockieren, und das Begrenzungselement (8) so mit dem Einstellelement (11) gekoppelt ist, dass sich das Begrenzungselement (8) beim Abgeben der eingestellten Dosis relativ zur Dosiervorrichtung zurück in die Ausgangsposition bewegt,
mit einer Rückdrehsicherung (7) für das Vorschubelement (9), um eine Drehung des Vorschubelementes (9) in eine Richtung zu ermöglichen und in Gegenrichtung zu sperren,
und
wobei als Vorschubelement (9) eine Gewindestange dient und die Dosiervorrichtung weiter eine weitere Gewindeführung für die Gewindestange umfasst,
so dass eine Axialbewegung des Betätigungs- oder Einstellelements (11) größer ist als eine Axialbewegung des Vorschubelements (9) oder untersetzt oder übersetzt wird, **dadurch gekennzeichnet, dass** die Rückdrehsicherung (7) axial verschiebesicher mit dem Begrenzungselement (8) gekoppelt ist, so dass das Begrenzungselement (8) die Rückdrehsicherung bei einer Bewegung in axialer Richtung mitnimmt und eine relative Drehung zwischen Begrenzungselement (8) und Rückdrehsicherung (7) möglich ist, so dass bei dieser relativen Drehung Klickgeräusche erzeugt werden.

2. Dosiervorrichtung nach Anspruch 1, wobei die Kupplung (12) so ausgelegt ist, dass das Betätigungs- oder Einstellelement (11) verdrehsicher mit dem Vorschubelement (9) gekoppelt werden kann.

3. Dosiervorrichtung nach einem der vorhergehenden Ansprüche, wobei die Kupplung ein Kupplungselement (12) ist, welches verdrehsicher mit dem Vorschubelement (9) oder dem Betätigungs- oder Einstellelement (11) gekoppelt ist und verdrehsicher mit dem Betätigungs- oder Einstellelement (11) oder mit dem Vorschubelement (9) gekoppelt werden kann.

4. Dosiervorrichtung nach Anspruch 3, wobei der Kupplungsmechanismus zum verdrehsicheren Koppeln des Kupplungselementes (12) mit dem Vorschubelement (9) oder dem Betätigungs- oder Einstellelement (11) eine Klauenkupplung ist, welche durch eine axiale Verschiebung des Kupplungselementes (12) relativ zum Vorschubelement (9) und/oder relativ zum Betätigungs- oder Einstellelement (11) eingekoppelt oder geöffnet werden kann.

5. Dosiervorrichtung nach einem der vorhergehenden Ansprüche mit einem Auslöseknopf (13), welcher drehbar an oder in dem Kupplungselement (12) und/oder dem Betätigungs- oder Einstellelement (11) gelagert ist.

6. Injektionsvorrichtung mit einer Dosiervorrichtung nach einem der vorhergehenden Ansprüche.

7. Dosiervorrichtung nach einem der vorhergehenden Ansprüche, wobei die Begrenzung oder der Anschlag ein Ende eines Gewindes einer Gewindestange (9) und/oder ein Axialanschlag und/oder ein Radialanschlag an der Gewindestange (9) oder einem Gehäuse (6) oder einem anderen Element (11, 12, 7) der Dosiervorrichtung ist.

8. Dosiervorrichtung nach einem der vorhergehenden Ansprüche, wobei das Begrenzungselement (8) auf der Gewindestange (9) gewindegeführt ist.

9. Dosiervorrichtung nach einem der vorhergehenden Ansprüche, wobei das Begrenzungselement (8) mit dem Betätigungs- oder Einstellelement (11) verdrehgesichert ist oder verdrehgesichert werden kann.

## Claims

1. A dose setting device for an injection device comprising
an actuating or adjusting element (11) for adjusting and dispensing a dose from an injection device,
an advance element (9) for producing an advance movement for dispensing a dose,
a coupling (12) with which the actuating or adjusting element (11) and the advance element (9) can be coupled for delivery of the dose adjusted with the actuating or adjusting element (11) in such a way that a rotary movement or dispensing movement of the actuating or adjusting element (11) is transmitted directly to the advance element (9),
a restricting element (8) for restricting the overall dose which can be adjusted at a maximum in an injection device, wherein the actuating or adjusting element (11) is so coupled to the restricting element (8) that the restricting element (8) moves in a movement of the actuating or adjusting element (11) from a starting position within the dose setting device in a direction towards a restriction or an abutment for blocking the movement of the restricting element (8) in order thereby to block the movement of the actuating or adjusting element (11) in an adjusting direction and the restricting element (8) is so coupled to the adjusting element (11) that the restricting element (8) moves back into the starting position relative to the dose setting device upon delivery of the adjusted dose,
a reverse rotation-preventing means (7) for the advance element (9) to permit rotation of the advance element (9) in one direction and to block it in the opposite direction, and
wherein a threaded rod serves as the advance element (9) and the dose setting device further includes a further thread guide for the threaded rod,
so that an axial movement of the actuating or adjusting element (11) is greater than an axial movement of the advance element (9) or is stepped up or stepped down, **characterised in that** the reverse rotation-preventing means (7) is axially non-displaceably coupled to the restricting element (8) so that the restricting element (8) entrains the reverse rotation-preventing means (7) in a movement in the axial direction and a relative rotation between the restricting element (8) and the reverse rotation-preventing means (7) is possible so that clicking noises are produced **in that** relative rotation.

2. A dose setting device according to claim 1 wherein the coupling (12) is so designed that the actuating or adjusting element (11) can be non-rotatably coupled to the advance element (9).

3. A dose setting device according to one of the preceding claims wherein the coupling is a coupling element (12) which is non-rotatably coupled to the advance element (9) or the actuating or adjusting element (11) and can be non-displaceably coupled to the actuating or adjusting element (11) or to the advance element (9).

4. A dose setting device according to claim 3 wherein the coupling mechanism for non-rotatable coupling of the coupling element (12) to the advance element (9) or the actuating or adjusting element (11) is a claw coupling which can be closed or opened by an axial displacement of the coupling element (12) relative to the advance element (9) and/or relative to the actuating or adjusting element (11).

5. A dose setting device according to one of the preceding claims comprising a triggering knob (13) mounted rotatably on or in the coupling element (12) and/or the actuating or adjusting element (11).

6. An injection device comprising a dose setting device according to one of the preceding claims.

7. A dose setting device according to one of the preceding claims wherein the restriction or the abutment is an end of a thread of a threaded rod (9) and/or an axial abutment and/or a radial abutment on the threaded rod (9) or a housing (6) or another element (11, 12, 7) of the dose setting device.

8. A dose setting device according to one of the preceding claims wherein the restricting element (8) is guided with a thread on the threaded rod (9).

9. A dose setting device according to one of the preceding claims wherein the restricting element (8) is or can be non-rotatably locked to the actuating or adjusting element (11).

## Revendications

1. Dispositif de dosage pour un dispositif d'injection,
avec un élément d'actionnement ou d'ajustement (11) pour l'ajustement et l'administration d'une dose à partir d'un dispositif d'injection,
avec un élément d'avancée (9) pour générer un mouvement d'avancée pour distribuer une dose,
avec un couplage (12) par l'intermédiaire duquel l'élément d'actionnement ou d'ajustement (11) et l'élément d'avancée (9) sont couplés pour l'administration de la dose ajustée avec l'élément d'actionnement ou d'ajustement (11), de telle sorte qu'un mouvement de rotation ou qu'un mouvement de distribution de l'élément d'actionnement ou d'ajustement (11) est transmis directement à l'élément d'avancée (9),
avec un élément de limitation (8) pour limiter une dose totale ajustable maximale dans un dispositif d'injection, dans lequel l'élément d'actionnement ou d'ajustement (11) est couplé avec l'élément de limitation (8) de telle sorte que l'élément de limitation (8) se déplace lors d'un mouvement de l'élément d'actionnement ou d'ajustement (11) d'une position de départ à l'intérieur du dispositif de dosage en direction d'une limitation ou d'une butée pour empêcher le mouvement de l'élément de limitation (8), pour bloquer ainsi le mouvement de l'élément d'actionnement ou d'ajustement (11) dans une direction d'ajustement, et l'élément de limitation (8) est couplé avec l'élément d'ajustement (11) de telle sorte que l'élément de limitation (8) revienne à la position de départ lors de l'administration de la dose ajustée par rapport au dispositif de dosage,
avec une sécurité empêchant une rotation inverse (7) pour l'élément d'avancée (9) afin de permettre une rotation de l'élément d'avancée (9) dans une direction et l'empêcher en sens inverse,
et
dans lequel une tige filetée sert d'élément d'avancée (9) et le dispositif de dosage comprend en outre un autre guidage fileté pour la tige filetée,
de sorte qu'un mouvement axial de l'élément d'actionnement ou d'ajustement (11) est plus grand qu'un mouvement axial de l'élément d'avancée (9) ou est diminué ou augmenté, **caractérisé en ce que**
la sécurité empêchant une rotation inverse (7) est couplée axialement sans risque de déplacement avec l'élément de limitation (8) de sorte que l'élément de limitation (8) entraîne la sécurité empêchant une rotation inverse lors d'un mouvement en direction axiale, et une rotation relative entre l'élément de limitation (8) et la sécurité empêchant une rotation inverse (7) est possible, ce qui permet de générer un clic lors de cette rotation relative.

2. Dispositif de dosage selon la revendication 1, dans lequel le couplage (12) est conçu de telle sorte que l'élément d'actionnement ou d'ajustement (11) peut être couplé sans risque de torsion avec l'élément d'avancée (9).

3. Dispositif de dosage selon l'une quelconque des revendications précédentes, dans lequel le couplage est un élément de couplage (12) qui est couplé sans risque de torsion avec l'élément d'avancée (9) ou l'élément d'actionnement ou d'ajustement (11) et peut être couplé sans risque de torsion avec l'élément d'actionnement ou d'ajustement (11) ou avec l'élément d'avancée (9).

4. Dispositif de dosage selon la revendication 3, dans lequel le mécanisme de couplage pour le couplage sans risque de torsion de l'élément de couplage (12) avec l'élément d'avancée (9) ou l'élément d'actionnement ou d'ajustement (11) est un couplage à griffes qui peut être couplé ou ouvert par un déplacement axial de l'élément de couplage (12) par rapport à l'élément d'avancée (9) et/ou par rapport à l'élément d'actionnement ou d'ajustement (11).

5. Dispositif de dosage selon l'une quelconque des revendications précédentes avec un bouton de déclenchement (13) qui est placé de façon à pouvoir tourner sur ou dans l'élément de couplage (12) et/ou l'élément d'actionnement ou d'ajustement (11).

6. Dispositif d'injection avec un dispositif de dosage selon l'une quelconque des revendications précédentes.

7. Dispositif de dosage selon l'une quelconque des revendications précédentes, dans lequel la limitation ou la butée est une extrémité d'un filetage d'une tige filetée (9) et/ou une butée axiale et/ou une butée radiale sur la tige filetée (9) ou un boîtier (6) ou un autre élément (11, 12, 7) du dispositif de dosage.

8. Dispositif de dosage selon l'une quelconque des revendications précédentes, dans lequel l'élément de limitation (8) est conduit par filetage sur la tige filetée (9).

9. Dispositif de dosage selon l'une quelconque des revendications précédentes, dans lequel l'élément de limitation (8) est protégé contre une torsion ou peut être protégé contre une torsion avec l'élément d'actionnement ou d'ajustement (11).
